# EUROPEAN PATENT SPECIFICATION

(11) **EP 3 922 294 B1**
(45) Date of publication and mention of the grant of the patent: **11.03.2026**
(21) Application number: 19914521.0
(22) Date of filing: 06.02.2019
(51) Int. Cl.: A61M 25/09, C25D 11/00, H01G 9/00

(54) **GUIDE WIRE**
FÜHRUNGSDRAHT
FIL-GUIDE

(43) Date of publication of application: 15.12.2021
(73) Proprietor: ASAHI INTECC CO., LTD., Seto-shi, Aichi 489-0071 (JP)
(72) Inventor: KUSANO, Yasuhiro, Seto-shi, Aichi 489-0071 (JP); KATAOKA, Maiko, Seto-shi, Aichi 489-0071 (JP); SHINOHARA, Yu, Seto-shi, Aichi 489-0071 (JP)
(74) Representative: Prinz & Partner mbB
(86) International application number: PCT/JP2019/004268
(87) International publication number: WO 2020/161832

(56) References cited:
- WO-A1-2010/058552
- WO-A1-2016/047555
- JP-A- 2017 133 101
- JP-A- 2017 153 615

## Description

The present invention relates to a guide wire.

For example, when treating an intravascular occluded site (e.g. chronic total occlusion: CTO) or the like caused due to progressive calcification, a guide wire for guiding therapeutic implements is inserted into a blood vessel prior to insertion of a therapeutic implement such as a balloon catheter.

Such a guide wire is demanded to have such an excellent shape restorability that a bent state is restored to an original shape state when the guide wire is bent by bringing the distal end of the guide wire into contact with an occluded site in a blood vessel. In addition, since the guide wire needs to be directed in a specific blood vessel direction at a site where the blood vessel is branched, the guide wire is also demanded to be easy to form such that a distal end portion of the guide wire can be previously bent into a desired shape.

Among these properties, in relation to the ease of forming, for example, there is a known technique in which a transverse section orthogonal to the longitudinal direction of a core shaft in the guide wire is formed into a flat shape as shown in JP 2016 067385 A. Such a technique is excellent in that the core shaft can be easily bent in a direction perpendicular to the flat direction.

In JP 2017 153615 A a guide wire with a tip portion having a smaller diameter than the base portion is shown. The tip has been heat modified with one portion having experienced a stronger heat treatment.

An electrode body for a capacitor is shown in WO 2010/058552 A1. The capacitor is made out of different electrode layers which combine to form a mesh network. The positive electrode substrates are formed from a NiTi alloy and the dieectric layers consist of titanium oxide.

JP 2017 133101 A describes a method for producing a Ni-Ti alloy having a surface layer which contains substantially no Ni. This is achieved by anodizing the Ni-Ti alloy in an aqueous solution.

WO 2016/047555 A1 further shows a guide wire with an elongated shape. The guide wire comprises a reshaping section with layers configured from different materials and their joined interface runs along a longitudinal direction of the core.

Herein, when pushing the guide wire forward in a branched blood vessel, the distal end portion of the guide wire should be directed in a particular blood vessel direction, and this operation is conducted by rotating a proximal end of the guide wire.

However, when the distal end portion of the core shaft in the guide wire is formed into a flat shape as described above, the aforementioned rotation of the distal end portion does not promptly follow the rotation of the proximal end, and the distal end portion of the core shaft that has been difficult to rotate at the initial stage of the rotation suddenly begins to rotate in some cases. This phenomenon is called "repellence", and when this repellence occurs, operability of the guide wire is decreased, and blood vessel selectivity of the guide wire is significantly reduced.

The present invention has been made on the basis of the above circumstances, and an object of the present invention is to provide a guide wire in which the ease of forming the distal end portion can be enhanced while maintaining excellent overall shape restorability.

The object is achieved by a guide wire having a core shaft, in which
the core shaft has a body portion,
wherein the body portion contains a nickel-titanium-based alloy having a superelastic property as a main component,
the core shaft has a layered portion having an inner layer formed on a part of an outer peripheral face of the body portion and containing a nickel alloy as a main component, and an outer layer formed on the inner layer and containing a titanium oxide as a main component,
and the superelastic property in the layered portion disappeared.

The core shaft may include two or more layered portions that are separated from each other,
such that the two or more layered portions are arranged in line symmetry with each other while sandwiching the body portion therebetween in a sectional view orthogonal to an axial direction of the core shaft.

The layered portion may be disposed on only one side region of the outer periphery of the body portion in the sectional view orthogonal to the axial direction of the core shaft.

The nickel-titanium-based alloy may be a nickel-titanium alloy.

In this specification, the "main component" means a component accounting for the largest mole fraction among the contained components at the relevant site.

The present invention makes it possible to provide a guide wire in which the ease of forming the distal end portion can be enhanced while maintaining excellent overall shape restorability.
FIG. 1 is a schematic sectional view in an axial direction illustrating an embodiment of the present invention.
FIG. 2 is a schematic sectional view taken along line II-II in FIG. 1.
FIG. 3A is a schematic sectional view in a transverse direction illustrating a first modification example.
FIG. 3B is a schematic sectional view in a transverse direction illustrating a second modification example.
FIG. 3C is a schematic sectional view in a transverse direction illustrating a third modification example.
FIG. 4A is a schematic sectional view in an axial direction illustrating a fourth modification example.
FIG. 4B is a schematic sectional view in an axial direction illustrating a fifth modification example.
FIG. 5A illustrates an example of a mapping image of titanium (Ti) in a section including a layered portion of a core shaft.
FIG. 5B illustrates an example of a mapping image of nickel (Ni) in the section including the layered portion of the core shaft.
FIG. 5C is an SEM image presenting the mapping regions in FIG. 5A and FIG. 5B.

The guide wire has a core shaft, in which the core shaft includes a body portion and a layered portion, the body portion contains a nickel-titanium-based alloy having a superelastic property as a main component, and the layered portion has an inner layer formed on a part of an outer peripheral face of the body portion and containing a nickel alloy as a main component and an outer layer formed on the inner layer and containing a titanium oxide as a main component.

In this specification, the "distal end portion" of the guide wire (core shaft) means a distal end site excluding a proximal end of an object, e.g. a small diameter portion, a tapered portion, and the like in the core shaft.

Hereinafter, an embodiment of the present invention will be explained with reference to the figures, but the present invention is not limited only to the embodiments described in the figures.

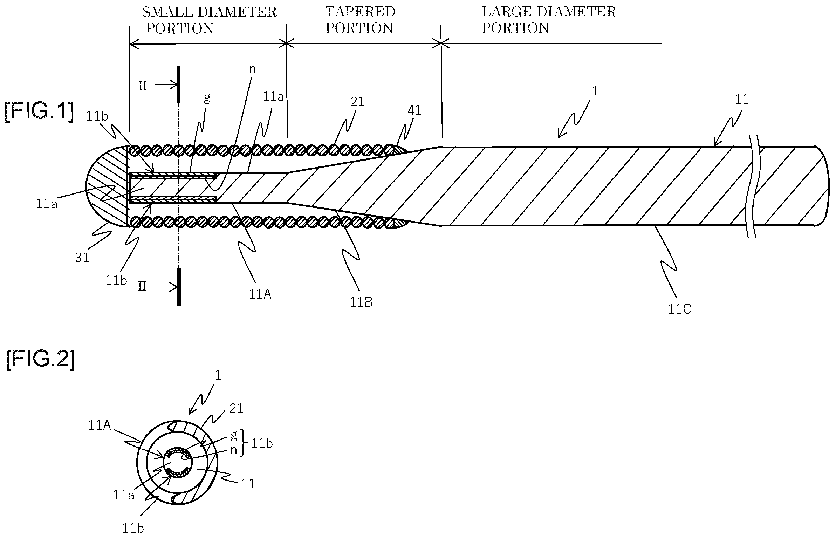

FIG. 1 is a schematic sectional view in an axial direction illustrating an embodiment of the present invention. FIG. 2 is a schematic sectional view taken along line II-II in FIG. 1. As illustrated in FIG. 1 and FIG. 2, a guide wire 1 is schematically composed of a core shaft 11, a coil body 21, and a distal end fixing part 31.

The core shaft 11 is a member constituting a central axis of the guide wire 1. The core shaft 11 can be formed e.g. such that the distal end portion thereof gradually decreases in diameter toward the distal end direction. In the present embodiment, the core shaft 11 is composed of a small diameter portion 11A, a tapered portion 11B, and a large diameter portion 11C in this order from the distal end. In a state that the guide wire 1 extends in a straight line, the small diameter portion 11A has a cylindrical shape, the large diameter portion 11C has a cylindrical shape with an outer diameter larger than the small diameter portion 11A, and the tapered portion 11B has a truncated cone shape that is continuous with the small diameter portion 11A and the large diameter portion 11C and gradually increases in diameter from the small diameter portion 11A to the large diameter portion 11C.

A total length of the core shaft 11 is typically 1,800 to 3,000 mm, preferably 1,800 to 2,500 mm. A length in the axial direction of the small diameter portion 11A is typically 0.5 to 50 mm, preferably 1 to 20 mm. A length in the axial direction of the tapered portion 11B is typically 10 to 200 mm, preferably 20 to 150 mm. An outer diameter of the small diameter portion 11A is typically 0.02 to 0.1 mm, preferably 0.03 to 0.07 mm. An outer diameter of the large diameter portion 11C is typically 0.25 to 1 mm, preferably 0.35 to 0.46 mm.

The present embodiment illustrates a guide wire in which the total length of the core shaft 11 is 1,900 mm, the length in the axial direction of the small diameter portion 11A is 10 mm, the length in the axial direction of the tapered portion 11B is 100 mm, the outer diameter of the small diameter portion 11A is 0.090 mm, and the outer diameter of the large diameter portion 11C is 0.335 mm.

The core shaft 11 includes a body portion 11a and a layered portion 11b.

The body portion 11a refers to a site containing a nickel-titanium-based alloy having a superelastic property as a main component in the core shaft 11.

Examples of the aforementioned nickel-titanium-based alloy include an Ni-Ti alloy (Ni=49 to 53 at%), an Ni-Ti-X alloy in which some of Ni atoms and/or Ti atoms in the Ni-Ti alloy are substituted with X atoms (X=Co, Fe, Mn, Cr, V, Al, Nb, W, B, etc., X=0.01 to 10 at%), an Ni-Ti-X alloy (X=Cu, Pb, or Zr, X=0.01 to 30 at%), and the like.

Among these alloys, the nickel-titanium alloy is preferable as the nickel-titanium-based alloy, and from the viewpoint of excellent superelastic property, an Ni-Ti alloy (Ni=49-53 at%) is more preferable. Thus, the core shaft 11 can acquire excellent shape restorability and high biocompatibility.

The layered portion 11b has an inner layer n and an outer layer g. The inner layer n refers to a site formed on a part on an outer peripheral face of the body portion 11a and containing a nickel alloy as a main component. This inner layer n is adjacent to the body portion 11a via a thin boundary layer (also referred to as "inner layer-body portion boundary layer") that is not illustrated, and a composition in the inner layer-body portion boundary layer continuously changes between a composition of the body portion 11a and a composition of the inner layer n. The outer layer g refers to a site formed on the inner layer n and containing a titanium oxide (e.g. titanium(IV) oxide: TiO₂, titanium(II) oxide: TiO, etc.) as a main component. An outer surface of this outer layer g is equivalent to an outer surface of the core shaft 11. In addition, the outer layer g is adjacent to the inner layer n via a thin boundary layer (also referred to as "inner layer-outer layer boundary layer") that is not illustrated, and a composition in the inner layer-outer layer boundary layer continuously changes between the composition of the inner layer n and a composition of the outer layer g.

Examples of the aforementioned nickel alloy include an alloy in which titanium atoms are excluded from the nickel-titanium-based alloy used as a base material, and the like. Specific examples include an Ni-Cu alloy as the nickel alloy in a case of using a Ni-Ti-Cu alloy as a base material, an Ni-Nb alloy as the nickel alloy in a case of using an Ni-Ti-Nb alloy as a base material, and the like.

In this embodiment, the layered portion 11b is formed only on a part of the small diameter portion 11A in the core shaft 11, and the body portion 11a is composed of the other part (site other than a part described above) of the small diameter portion 11A, the tapered portion 11B, and the large diameter portion 11C.

Herein, the core shaft 11 includes two or more layered portions that are separated from each other, and it is preferable that the two or more layered portions are arranged in line symmetry with each other while sandwiching the body portion therebetween in a sectional view orthogonal to an axial direction of the core shaft. In this embodiment, as illustrated in FIG. 2, the small diameter portion 11A of the core shaft 11 includes two layered portions 11b and 11b that are separated from each other, and the two layered portions 11b and 11b are arranged in line symmetry with each other while sandwiching the body portion 11a therebetween in a sectional view orthogonal to an axial direction of the small diameter portion 11A.

In this way, the core shaft 11 includes two layered portions 11b and 11b that are arranged in line symmetry with each other while sandwiching the body portion 11a therebetween, and thereby the core shaft 11 can be easily and reliably formed in a specific direction (in this embodiment, a direction from a central axis (body portion 11a) of the core shaft 11 toward the layered portion 11b side).

As the method of forming the layered portions 11b, for example, a method of heating a surface of the core shaft 11 on which the layered portions 11b are formed, by irradiating the surface with a laser light such as a YAG laser and a semiconductor laser (laser heating method), a method of heating the surface by bringing the surface into direct contact with a high-temperature heat source (direct heating method), and the like can be adopted. Among these methods, the laser heating method is preferable from the viewpoint that the layered portions 11b can accurately be formed only on a desired site in a short time. The region (area, depth) on which the layered portions 11b are formed is not particularly limited as long as the inner layer n is disposed on the outer peripheral face of the body portion 11a, and can be adjusted by appropriately selecting the heating conditions depending on a desired bent shape.

The coil body 21 is wound so as to cover at least a part of an outer periphery of the core shaft 11, and can be composed of e.g. a single-thread coil or the like obtained by spirally winding one solid wire such that adjacent wire rods are in contact with each other.

A diameter of a wire constituting the coil body 21 is typically 0.01 to 0.10 mm, preferably 0.01 to 0.08 mm. In the present embodiment, a single-thread coil body 21 obtained by spirally winding a wire having a diameter of 0.06 mm is described as an example.

As a material of the wire constituting the coil body 21, for example, a stainless steel such as SUS316; a superelastic alloy such as a Ni-Ti alloy; a radiopaque metal such as platinum and tungsten; or the like can be adopted.

For example, the aforementioned coil body 21 has a distal end fixed to the distal end fixing part 31 described below, and a proximal end fixed to the outer periphery of the core shaft 11 on a joint part 41. As a method of fixing the coil body 21 to the core shaft 11, for example, a brazing method or the like can be adopted. Examples of a brazing material used in the aforementioned brazing method include a brazing metal such as an Sn-Pb alloy, an Pb-Ag alloy, an Sn-Ag alloy, and an Au-Sn alloy, and the like.

The distal end fixing part 31 refers to a site where the distal end of the core shaft 11 and the distal end of the coil body 21 are fixed to each other. Specifically, in this distal end fixing part 31, for example, the distal end of the core shaft 11 and the distal end of the coil body 21 are integrally brazed. As for a shape of the distal end fixing part 31, so as not to damage an inner wall of a blood vessel when advancing the guide wire 1 in the blood vessel, for example, a brazing material can be used to form the distal end fixing part 31 into a hemispherical shape in which the distal end side portion of the distal end fixing part 31 is smoothly curved. Examples of the brazing material used for the distal end fixing part 31 includes the same brazing materials as those described for the brazing method of the coil body 21 and the core shaft 11 as an example, and the like.

Next, an example of a usage mode of the guide wire 1 will be explained. First, a surgeon bents the distal end portion of the guide wire 1 into e.g. a J-shape. At this time, a site to be bent in the guide wire 1 refers to the region where the layered portion 11b is formed in the axial direction of the core shaft 11. In this region, the guide wire 1 can be bent into any desired shape as long as the bending direction is orientated from the central axis of the core shaft 11 toward the layered portion 11b side.

Subsequently, the distal end of the guide wire 1 having the bent distal end portion is inserted into the blood vessel and then pushed toward a treatment site. At this time, for example, when the guide wire 1 reaches a branched site of the blood vessel, the distal end portion of the guide wire 1 is rotated as necessary. The surgeon rotates the proximal end of the guide wire 1 to enable this rotation of the distal end portion. After the guide wire 1 reaches a treatment site, an instrument such as a balloon catheter and a stent not illustrated is transported along the guide wire 1, to perform various treatments at the treatment site. After the treatment is completed, the guide wire 1 is retracted in the blood vessel and drawn out from the body, and the series of procedures is completed.

As described above, since the guide wire 1 has the aforementioned configuration, a local formability can be enhanced on the layered portion while maintaining excellent overall shape restorability by the superelastic property of the body portion 11a. It is inferred that this is because the superelastic property in the layered portion disappeared due to the denaturation of the base material in association with the thermal action, and as a result, plastic deformation became possible. As a result, the guide wire 1 makes it possible to improves operability and perform the procedure promptly and reliably.

Note that the present invention is not limited to the configurations of the aforementioned embodiments, but is stipulated by claims, and the present invention is intended to include all modifications within the meaning and scope to those in claims.

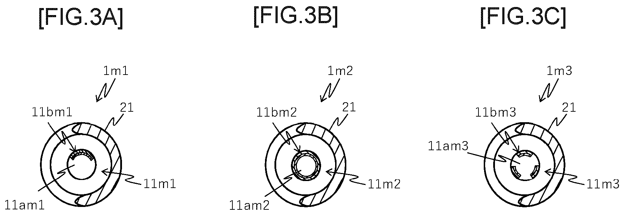

For example, in the aforementioned embodiments, although the guide wire 1 in which the two layered portions 11b and 11b of the core shaft 11 are arranged in line symmetry with each other while sandwiching the body portion 11a therebetween has been explained, the guide wire may be e.g. a guide wire 1m1 in which a layered portion 11bm1 is disposed only on one side region in an outer periphery of a body portion 11am1 in a sectional view orthogonal to an axial direction of a core shaft 11m1, as illustrated in FIG. 3A. Also, this makes it possible to easily and reliably form the core shaft in a specific direction in the same manner as in the aforementioned embodiments. In addition, the arrangement of the layered portion in the sectional view orthogonal to the axial direction of the core shaft may be represented by not only the guide wire 1m1 in FIG. 3A but also e.g. a guide wire 1m2 in which a layered portion 11bm2 is arranged over an entire periphery of a body portion 11am2 in a sectional view orthogonal to an axial direction of a core shaft 11m2 (see FIG. 3B), a guide wire 1m3 in which layered portions 11bm3 are independently disposed at three or more sites on an outer periphery of a body portion 11am3 in a sectional view orthogonal to an axial direction of a core shaft 11m3 (see FIG. 3C), or the like.

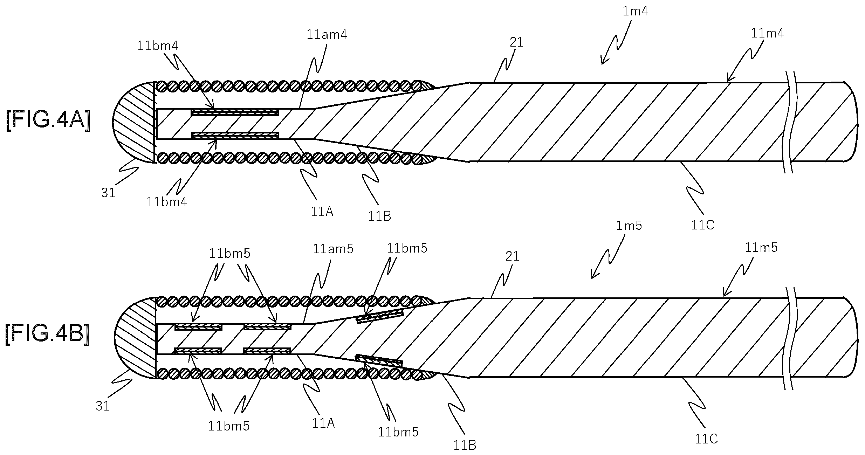

Additionally, in the aforementioned embodiment, as the site of layered portions 11b in the axial direction of the core shaft 11, the guide wire 1 in which the layered portions 11b are continuous with the distal end fixing part 31 and formed only on a part of the small diameter portion 11A (no layered portion is formed on the tapered portion 11B and the large diameter portion 11C) has been explained, but the layered portions may be formed on any site of the small diameter portion, the tapered portion, and the large diameter portion as long as the effects of the present invention is not impaired. The layered portions may be located at a plurality of sites in the axial direction of the core shaft. Examples of such a guide wire include a guide wire 1m4 in which layered portions 11bm4 are disposed only on the way of a core shaft 11m4 in an axial direction of a core shaft 11m4 (see FIG. 4A), a guide wire 1m5 in which layered portions 11bm5 are independently disposed at a plurality of sites in an axial direction of a core shaft 11m5 (see FIG. 4B), and the like.

In addition, in the aforementioned embodiments, although the guide wire 1 having the small diameter portion 11A, the tapered portion 11B, and the large diameter portion 11C has been explained, the guide wire may be a guide wire including no small diameter portion and/or tapered portion, or a guide wire including a core shaft having a distal end portion with another shape.
Also, in the aforementioned embodiment, although the guide wire 1 including the coil body 21 and the distal end fixing part 31 has been explained, the guide wire may be a guide wire including a coil body and a distal end fixing part that have other shapes, or a guide wire including no coil body and/or distal end fixing part.

### Examples

Hereinafter, the present invention will be explained with reference to Examples, but the present invention is not limited to Examples.

### <Production of Core Shaft>

### [Production Example 1]

Using an Ni-Ti alloy (Ni=51 at%) as a base material and by centerless polishing, a core shaft having a total length of 1,900 mm was formed, which has a small diameter portion (cylindrical shape, length in the axial direction: 10 mm, outer diameter: 0.090 mm), a tapered portion (truncated cone shape, length in the axial direction: 100 mm), and a large diameter portion (cylindrical shape, outer diameter: 0.335 mm) in this order from the distal end.

Subsequently, using the obtained core shaft, a laser light (fiber laser) is emitted to two surface regions within a range of 5 mm from the distal end toward the proximal end of the small diameter portion in this core shaft and opposite to each other with respect to the central axis of the core shaft, to obtain a core shaft including two layered portions separated from each other and arranged in line symmetry with each other while sandwiching a body portion therebetween in a sectional view orthogonal to an axial direction of the core shaft.

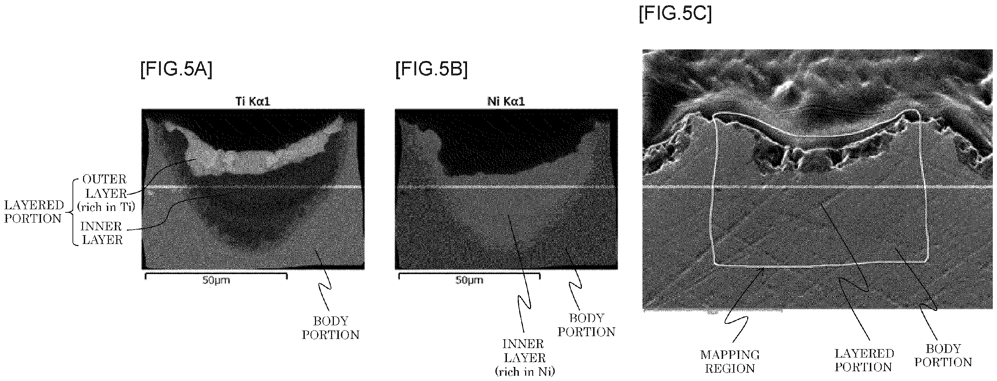

FIG. 5A to FIG. 5C present examples of mapping images of titanium (Ti) and nickel (Ni) in a section including the layered portion of the core shaft analyzed using an energy dispersive X-ray spectrometer (EDX, Energy dispersive X-ray Spectrometry, Model: AZtec Energy Advanced X-Max50, manufactured by Oxford Instruments) annexed to a field emission type scanning electron microscope (Model: SU-70, manufactured by Hitachi High-Tech Corporation.), and an SEM (scanning electron microscope) image (FIG. 5A is a mapping image of Ti, FIG. 5B is a mapping image of Ni, and FIG. 5C is a SEM image of the aforementioned section (the mapping area is inside the white line)). As can be seen from this mapping image, in Production Example 1, a part of the Ni-Ti alloy used as the base material disappeared by irradiating the surface of the core shaft with the laser light, and a layered portion composed of an outer layer containing Ti atoms and an inner layer without Ti atoms was formed on a part of the outer peripheral face of the body portion.

### <Production of Guide Wire>

### [Example 1]

A single-thread coil body (material: platinum and stainless steel, wire diameter: 0.06 mm, coil outer diameter: 0.345 mm, length: 110 mm) previously wound was used, and the core shaft produced in Production Example 1 was inserted into a central hole of the coil body. Then, the distal end of the coil body and the distal end of the core shaft were integrally brazed using a brazing material, to form a hemispherical distal end fixing part, and the proximal end of the coil body was brazed to the outer peripheral face of the tapered portion of the core shaft to form a joint part, so that a guide wire of Example 1 was obtained.

### REFERENCE SIGNS LIST

- 1, 1m1 to 1m5: Guide wire
- 11, 11m1 to 11m5: Core shaft
- 11a, 11am1 to 11am5: Body portion
- 11b, 11bm1 to 11bm5: Layered portion
- 21: Coil body
- 31: Distal end fixing part
- n: Inner layer
- g: Outer layer

## Claims

1. A guide wire (1; 1m1 - 1m5) having a core shaft (11; 11m1 - 11m5) comprising a body portion (11a; 11am1 - 11am5),
wherein the body portion (11a; 11am1 - 11am5) contains a nickel-titanium-based alloy having a superelastic property as a main component,
charcterised in that the core shaft (11; 11m1 - 11m5) further comprises a layered portion (11b; 11bm1 - 11bm5),
wherein the layered portion (11b; 11bm1 - 11bm5) has an inner layer (n) formed on a part of an outer peripheral face of the body portion (11a; 11am1 - 11am5) and containing a nickel alloy as a main component, and an outer layer (g) formed on the inner layer (n) and containing a titanium oxide as a main component,
and the superelastic property in the layered portion (11b; 11bm1 - 11bm5) disappeared.

2. The guide wire (1; 1m4, 1m5) according to claim 1, wherein
the core shaft (11; 11m4, 11m5) comprises two or more layered portions (11b; 11bm4, 11bm5) that are separated from each other, and
the two or more layered portions (11; 11bm4, 11bm5) are arranged in line symmetry with each other while sandwiching the body portion (11a; 11am4, 11am5) therebetween in a sectional view orthogonal to an axial direction of the core shaft (11; 11m4, 11m5).

3. The guide wire (1m1) according to claim 1, wherein the layered portion (11mb1) is disposed on only one side region of the outer periphery of the body portion (11am1) in the sectional view orthogonal to the axial direction of the core shaft (11m1).

4. The guide wire (1; 1m1 - 1m5) according to any one of claims 1 to 3, wherein the nickel-titanium-based alloy is a nickel-titanium alloy.

## Patentansprüche

1. Führungsdraht (1; 1m1 - 1m5) mit einem Kernschaft (11; 11m1 - 11m5), der einen Körperabschnitt (11a; 11am1 - 11am5) umfasst,
wobei der Körperabschnitt (11a; 11am1 - 11am5) eine Legierung auf Nickel-Titan-Basis mit einer superelastischen Eigenschaft als Hauptbestandteil enthält,
**dadurch gekennzeichnet, dass** der Kernschaft (11; 11m1 - 11m5) ferner einen geschichteten Abschnitt (11b; 11bm1 - 11bm5) umfasst,
wobei der geschichtete Abschnitt (11b; 11bm1 - 11bm5) eine Innenschicht (n), die auf einem Teil einer Außenumfangsfläche des Körperabschnitts (11a; 11am1 - 11am5) gebildet ist und eine Nickellegierung als Hauptbestandteil enthält, und eine Außenschicht (g) aufweist, die auf der Innenschicht (n) gebildet ist und ein Titanoxid als Hauptbestandteil enthält,
und die superelastische Eigenschaft in dem geschichteten Abschnitt (11b; 11bm1 - 11bm5) verschwunden ist.

2. Führungsdraht (1; 1m4, 1m5) nach Anspruch 1, bei dem
der Kernschaft (11; 11m4 - 11m5) zwei oder mehr geschichtete Abschnitte (11b; 11bm4, 11bm5) umfasst, die voneinander getrennt sind, und
die zwei oder mehr geschichteten Abschnitte (11b; 11bm4, 11bm5) in einer Schnittansicht orthogonal zu einer Axialrichtung des Kernschafts (11; 11m4, 11m5) in einer Achsensymmetrie zueinander angeordnet sind, während sie den Körperabschnitt (11a; 11am4, 11am5) zwischen sich einschließen.

3. Führungsdraht (1m1) nach Anspruch 1, bei dem der geschichtete Abschnitt (11mb1) in der Schnittansicht orthogonal zur Axialrichtung des Kernschafts (11m1) an nur einem Seitenbereich des Außenumfangs des Körperabschnitts (11am1) angeordnet ist.

4. Führungsdraht (1; 1m1 - 1m5) nach einem der Ansprüche 1 bis 3, bei dem die Legierung auf Nickel-Titan-Basis eine Nickel-Titan-Legierung ist.

## Revendications

1. Fil de guidage (1 ; 1m1 - 1m5) présentant une tige médiane (11 ; 11m1 - 11m5) comprenant une partie de corps (11a ; 11am1 - 11am5),
la partie de corps (11a ; 11am1 - 11am5) contenant un alliage à base de nickel et de titane à propriété superélastique comme composant principal,
**caractérisé en ce que** la tige médiane (11 ; 11m1 - 11m5) comprend en outre une partie en couches (11b ; 11bm1 - 11bm5),
la partie en couches (11b ; 1 1bm1 - 11bm5) comportant une couche intérieure (n) formée sur une partie d'une face périphérique extérieure de la partie de corps (11a ; 11am1 - 11am5) et contenant un alliage de nickel comme composant principal, et une couche extérieure (g) formée sur la couche intérieure (n) et contenant un oxyde de titane comme composant principal,
et la propriété superélastique dans la partie en couches (11b ; 11bm1 - 11bm5) ayant disparu.

2. Fil de guidage (1 ; 1m4, 1m5) selon la revendication 1, la tige médiane (11 ; 11m4, 11m5) comprenant deux parties en couches (11b ; 11bm4, 11bm5) ou plus qui sont séparées les unes des autres, et
les deux parties en couches (11 ; 11bm4, 11bm5) ou plus étant agencées en symétrie linéaire les unes par rapport aux autres tout en prenant en sandwich la partie de corps (11a ; 1 1am4, 11am5) entre elles dans une vue en coupe orthogonale à un sens axial de la tige médiane (11 ; 11m4, 11m5).

3. Fil de guidage (1m1) selon la revendication 1, la partie en couches (11mb1) étant agencée uniquement sur une zone latérale de la périphérie extérieure de la partie de corps (11am1) dans la vue en coupe orthogonale au sens axial de la tige médiane (11m1).

4. Fil de guidage (1 ; 1m1 - 1m5) selon l'une des revendications 1 à 3, l'alliage à base de nickel et de titane étant un alliage nickel-titane.
